# EUROPÄISCHE PATENTSCHRIFT

(11) **EP 0 257 572 B1**
(45) Veröffentlichungstag und Bekanntmachung des Hinweises auf die Patenterteilung: **21.12.1994**
(21) Anmeldenummer: 87112127.3
(22) Anmeldetag: 21.08.1987
(51) Int. Cl.: C08G 65/32, C07C 269/04, G01N 33/53

(54) **Neue Thyroninderivate**
Thyronin derivatives
Dérivés de thyronine

(30) Priorität: 25.08.1986 DE 3628795
(43) Veröffentlichungstag der Anmeldung: 02.03.1988
(73) Patentinhaber: HOECHST AKTIENGESELLSCHAFT, 65926 Frankfurt am Main (DE)
(72) Erfinder: Wissmann, Hans, Dr., D-6232 Bad Soden am Taunus (DE); Simons, Guido, Dr., D-6507 Ingelheim am Rhein (DE); Strecker, Helmut, Dr., verstorben (DE)

(56) Entgegenhaltungen:
- EP-A- 0 108 400
- EP-A- 0 108 403
- EP-A- 0 190 765
- JOURNAL OF MACROMOLECULAR SCIENCE; REVIEWS IN MACROMOLECULAR CHEMISTRY AND PHYSICS, Band C25, Nr. 3, 1985, Seiten 325-373, New York, US; J.M. HARRIS; "Laboratory synthesis of polyethylene glycol derivatives"
- MAKROMOLEKULARE CHEMIE; MACROMOLECULAR AND PHYSICS, Band 187, Nr. 5, Mai 1986, Seiten 1131-1144, Heidelberg, DE; K. ULBRICH et al.: "Poly(ethylene glycol)s containing enzymatically degradable bonds"

## Beschreibung

Die Erfindung betrifft neue Thyroninderivate der allgemeinen Formel I 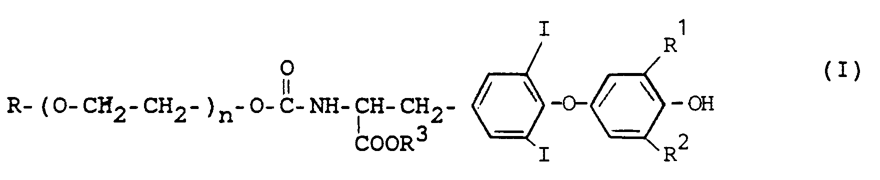
in welcher
- R: Wasserstoff, (C₁-C₆)-Alkyl oder eine mit chemischen oder physikalischen Methoden quantifizierbare Gruppe, wie z.B. einen Rest der Formel 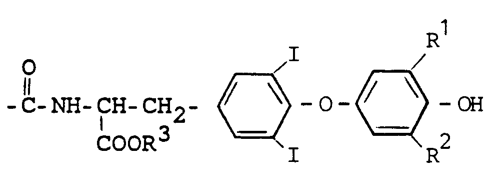 bedeutet,
- R¹ und R²: gleich oder verschieden sind und Iod oder Wasserstoff bedeuten,
- R³: Wasserstoff oder (C₁-C₆)-Alkyl bedeutet und
- n: für eine ganze Zahl zwischen 10 und 400 steht,
sowie deren physiologisch verträglichen Salze mit Kationen.

Bevorzugt wird Verbindungen der Formel I in denen R¹ und R² wie oben definiert sind und n = 10 -140 insbesondere 35 - 70 ist, R (C₁-C₄)-Alkyl, insbesondere Methyl oder Wasserstoff, R³ Wasserstoff oder (C₁-C₄)-Alkyl, insbesondere Methyl oder Ethyl, bedeuten und in welchen mindestens eines der Iodatome radioaktiv markiert ist.

Unter Salzen der Verbindungen der Formel I werden insbesondere Alkalimetall-, Erdalkalimetall- und Ammoniumsalze verstanden.

Unter einer mit chemischen oder physikalischen Methoden quantifizierbaren Gruppe versteht man einen organischen Rest, der bei einem Immunoassay zur Markierung dient. Ein solcher Rest kann fluoreszierend, lumineszierend, elektroaktiv, spin labelled oder radioaktiv markiert sein (vgl. Neumüller, Römpps Chemie Lexikon, 8. Auflage, Stuttgart 1983; Gunzer, Rieke, Kontakte Merck 1980, Nr. 3, 3-11; Eckert, Angew. Chem. 88 [1976] 565-574).

Die Erfindung betrifft weiterhin ein Verfahren zur Herstellung von Verbindungen der Formel I, das dadurch gekennzeichnet ist, daß man Verbindungen der allgemeinen Formel II,

R-(O-CH₂-CH₂-)ₙ-OH (II)

in welcher R und n wie oben definiert sind, die freie alkoholische OH-Gruppe entweder als Kohlensäureesterchlorid oder als mit geeigneten Aktivestergruppen substituierten Ester der Kohlensäure mit einer Verbindung der Formel III 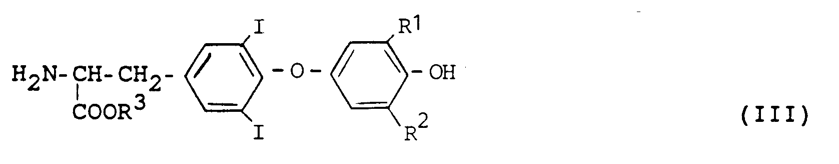
in welcher R¹ und R² wie oben definiert sind und R³ Wasserstoff bedeutet, umsetzt, oder gegebenenfalls einen Ester der Formel III (R³ ‡ H) einsetzt, erhaltene Ester der Formel I (R³ ‡ H) gegebenenfalls in die freien Säuren der Formel I (R³ = H) überführt, und gegebenenfalls die so erhaltene Verbindung der Formel I in ihre Salze überführt.

Vorzugsweise behandelt man die oben genannten Verbindungen der Formel II mit Phosgen in inerten, wasserfreien Lösungsmitteln (z.B. Chlorkohlenwasserstoffen) analog dem in Houben-Weyl, Methoden der Organischen Chemie (Georg Thieme Verlag, Stuttgart, 1952 Band 8/III S.103) für die Umsetzung niedermolekularer Verbindungen von W. Krey beschriebenen Verfahren. Die so entstandenen Carbonylchloridderivate werden dann mit den beschriebenen Di-, Tri- oder Tetraiodthyroninderivaten der allgemeinen Formel III umgesetzt.

Anstelle der Chloride können auch noch andere aktivierte Derivate der Kohlensäure, z.B. die N-Hydroxy-succinimidester, Pentachlorphenylester, Nitrophenylester und ähnliche verwendet werden.

Die Umsetzung der oben genannten Kohlensäurederivate mit Verbindungen der Formel III findet in gemischtwäßrigem Medium bei einem pH von 7-10, vorzugsweise 8,5-10 statt. Als Lösungsmittel werden Dimethylformamid/Wasser, oder Dimethylacetamid/Wasser verwendet. Nicht umgesetztes Thyroninderivat der Formel III wird durch Dialyse, Sephadex-Chromatographie oder Ultrafiltration z.B. über eine UM 2-Membran (Fa. Amicon) abgetrennt.

Bedingt durch die Herstellungsweise fallen in der Regel Gemische von zwei oder mehreren Verbindungen der Formel I an. Die Erfindung betrifft daher auch Zubereitungen, die zwei oder mehrere Verbindungen der Formel I enthalten.

Die Erfindung betrifft weiterhin die Verwendung von Verbindungen der Formel I oder deren Gemischen bei der Durchführung eines Immunoassays, vorzugsweise eines Radioimmunoassays.

Nachstehende Beispiele dienen zur Erläuterung der vorliegenden Erfindung, ohne daß diese darauf beschränkt wäre.

### Beispiel 1:

### Polyethylenglycol-bis-oxycarbonyl-L-3,3ʹ,5-triiod-thyronin

220 mg (0,3 mMol) L-Triiodthyronin löst man unter Zugabe von wäßriger 2n NaOH am Autotitrator bei pH 10 unter Rühren in einer Mischung aus 4 ml Dimethylformamid und 4 ml Wasser. Dann gibt man DMF und anschließend portionsweise innerhalb von 1 Stunde 1 g Polyethylenglycol-bis-oxycarbonylchlorid vom Molgewicht 6000 zu. Man läßt 12 Stunden bei Raumtemperatur und pH 9,5 nachreagieren, filtriert von einer kleinen Menge an schwerlöslichem Material ab, bringt dann die Lösung im Hochvakuum bei Raumtemperatur zur Trockne, nimmt in 100 ml Wasser auf und säuert mit wäßriger 1n-HCl auf pH 3 an. Man dialysiert die so erhaltene Lösung gegen insgesamt 30 l Wasser und isoliert das Endprodukt durch Gefriertrocknung. Der so erhaltene, schwachgelbliche Festkörper zeigt einen Iodgehalt von 11 %, was einem Gehalt an gebundenem Triiodthyronin von ca. 1,7 Mol/Mol Thyroninderivat entspricht.
Ausbeute: 975 mg

### Beispiel 2:

### Monomethyl-polyethylenglycolyloxycarbonyl-L-3,5-diiodthyroninverbindung

940 mg (2 mMol) L-Diiodthyronin löst man in einem Gemisch aus 6 ml Dimethylformamid und 3 ml Wasser bei pH 8,8 - 9,5 am Autotitrator. Dann gibt man portionsweise innerhalb von 1 Stunde 1 g Monomethyl-polyethylenglycoloxy-carbonylchlorid (Molgewicht 750) zu. Nach 30 Minuten Nachrühren bei Raumtemperatur wird nochmals 1 g des vorstehend beschriebenen Carbonylchlorids zugegeben. Nach Rühren über Nacht ist die Lösung nahezu klar. Man dampft die Lösungsmittel im Hochvakuum bei Raumtemperatur ab und nimmt den Rückstand in 30 ml Wasser auf, säuert mit verdünnter Salzsäure auf pH 3 an, extrahiert die Wasserphase mehrfach mit Methylenchlorid, trocknet die Methylenchloridphase über Magnesiumsulfat, und erhält nach dem Abdestillieren ein farbloses Öl, das laut Elementaranalyse (C,H,I) ca. 30 % der Monomethylpolyethylenglykol-diiodthyroninverbindung enthält.
Ausbeute: 2,5 g.

Die weitere Reinigung erfolgt durch Säulenchromatographie an ^{(R)}Sephadex G 10 in wäßriger 0,1 m Essigsäure.
- Ausbeute:: 0,5 g eines laut Elementaranalyse 80 % igen Präparates.

### Beispiel 3:

### Monomethyl-polyethylenglycolyloxycarbonyl-L-3,3ʹ, 5,5ʹ-tetraiodthyroninverbindung

350 mg L-3,3ʹ, 5,5ʹ-Tetraiodthyronin löst man durch Zugabe von wäßriger 0,5 n Natronlauge bei pH 9,5 in einem Gemisch aus 4 ml Dimethylformamid und 4 ml Wasser. In diese Lösung läßt man unter Rühren die Lösung von 1 g Polyethylenglycol-methylether-carbonylchlorid (Molgewicht ca. 1900) in 4 ml Dimethylformamid und Konstanthalten des pH bei 9,5 innerhalb von einer Stunde zutropfen. Während der Zugabe des Säurechlorids klärt sich die anfänglich entstandene Suspension wieder. Nach Stehenlassen über Nacht säuert man mit verdünnter Salzsäure auf pH 3 an, und destilliert die Lösungsmittel im Hochvakuum bei Raumtemperatur ab. Man trocknet den Rückstand im Exsiccator, und kocht ihn anschließend dreimal mit absolutem Ether aus. Dann nimmt man ihn in 16 ml siedendem Methylchlorid auf, filtriert vom Ungelösten und läßt dann im Eisbad abkühlen.

Der ausgefallene Festkörper wird abgesaugt: 565 mg. Durch Einengen der Mutterlauge gewinnt man eine Restfraktion: 200 mg. Beide Fraktionen zeigen identische und von 3,3ʹ,5,5ʹ -Tetraiodthyronin verschiedene Wanderungsstrecken im DC-System Chloroform/Methanol/20 % Ameisensäure (69:30:7,5) und identische Elementaranalysenbefunde, die auf etwa 1 Mol gebundenes 3,3ʹ,5,5ʹ-Tetrajodthyronin/Mol der Verbindung hinweisen.

### Beispiel 4

### Polyethylenglycolyloxycarbonyl-diiodthyroninverbindung

470 mg 3,3-Diiodthyronin und 2,2 g Polyethylenglycol-bis-oxycarbonylchlorid (Molgewicht 6000) setzt man wie unter Beispiel 2 beschrieben um, arbeitet jedoch bereits nach einstündiger Reaktionszeit auf. Man erhält 2,8 g eines farblosen Festkörpers von wachsartiger Konsistenz, der laut Elementaranalyse (Iod,C,H) ca. 80 % des mit 1 Mol 3,3-Diiodthyronin substituierten Polymeren enthält. Die Dünnschichtchromatographie, wie im Beispiel 3 beschrieben, zeigt die Verschiedenheit des Reaktionsproduktes vom Ausgangsmaterial.

### Beispiel 5:

### Monomethyl-polyethylenglycolyloxycarbonyl-L-3,5-diiodthyroninverbindung

Die Verbindung wird wie im Beispiel 2 beschrieben, aber unter Verwendung des Carbonylchlorids eines Monomethyl-polyethylenglycols vom Molgewicht 3000 hergestellt. Das Rohprodukt enthält laut Elementaranalyse 50 % der Titelverbindung.
Ausbeute: 3,1 g farblose halbfeste Substanz.

### Beispiel 6:

### Polyethylenglycoloxycarbonyl-L-3,3ʹ,5-Triiodthyroninmethylester

Die Verbindung wurde wie unter Beispiel 1 beschrieben, aber unter Verwendung von L-Triiodthyroninmethylester anstelle von L-Triiodthyronin und unter Verwendung eines Polyethylenglycol-bis-oxycarbonylchlorids vom Molgewicht 15 000 hergestellt. Die Reaktionszeit wurde auf 2 Stunden verkürzt. Das entstandene Reaktionsprodukt zeigte laut Elementaranalyse einen Einbau von einem Mol L-Triiodthyroninmethylester/Mol Polyethylenglykol.
Ausbeute bei gleicher Ansatsgewichtsmenge wie im Beispiel 1: 750 mg

### Beispiel 7:

### Arbeitsvorschrift zur Bestimmung von FT4 nach dem 2-Schritt-Verfahren (vgl. hierzu Eckert, Angew. Chem. 88 [1976] 565-574)

In Röhrchen, die mit T4-Antikörper beschichtet sind (20 ng Ak/pro Röhrchen) werden 200 µl einer Standardreihe (Humanseren mit steigendem FT4-Gehalt) und 1000 µl Puffer eine halbe Stunde unter Schütteln in Kontakt gebracht.

Nach dem Ausschütten der Reaktionslösung werden 1000 µl des aus der Verbindung des Beispiels 3 hergestellten Tracers (Aktivität ca. 60 000 Impulse pro Minute) in das vorinkubierte Röhrchen gegeben. Es wird eine Stunde inkubiert, der nicht gebundene Tracer abgetrennt und in einem γ-Zähler gemessen.

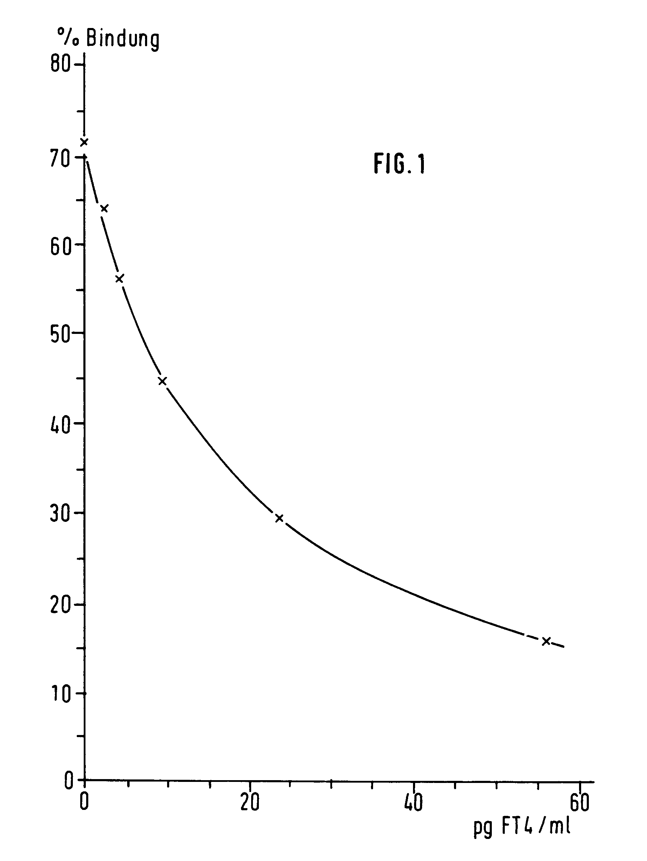

Figur 1 zeigt den Standardverlauf von Humanseren mit steigendem FT4 -Gehalt. Der Tracer wurde aus dem im Beispiel 3 beschriebenen Derivat hergestellt.

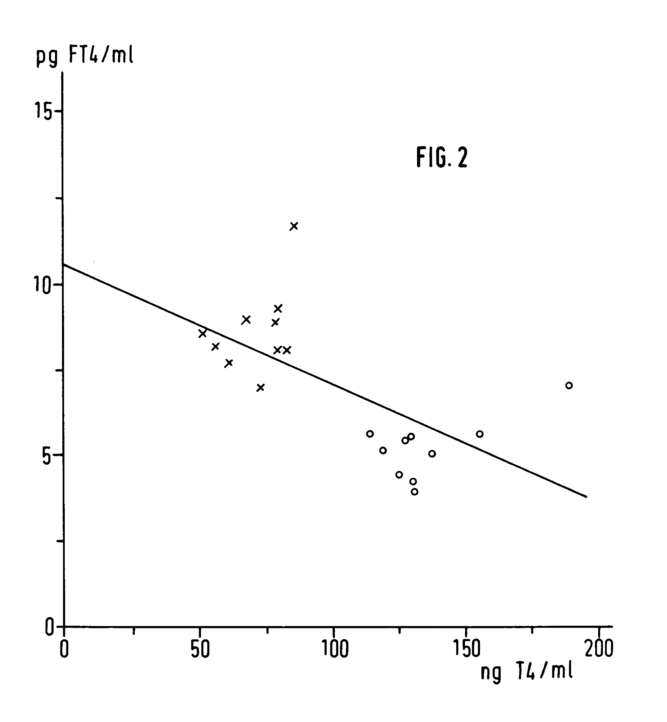

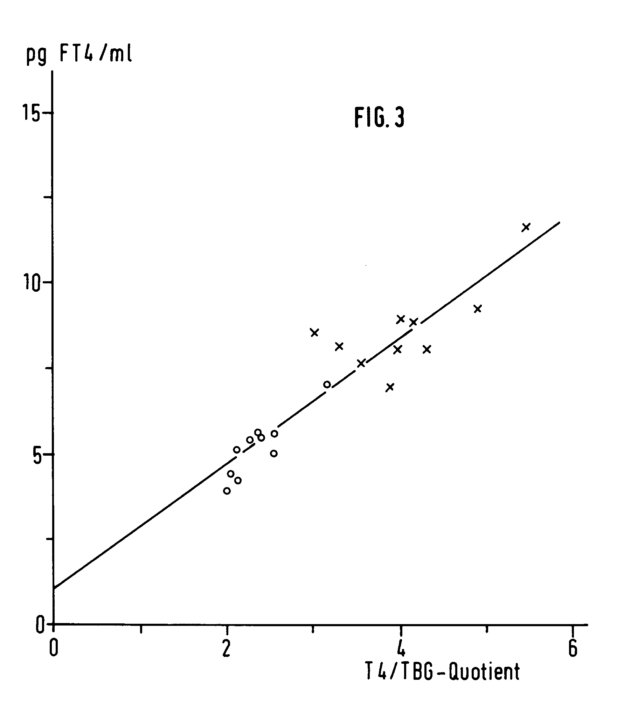

Fig. 2 und 3 zeigen Ergebnisse der Untersuchung von Humanseren (als "*" dargestellt). Die Seren schwangerer Frauen (als "o" dargestellt) wurden für die Untersuchung mit herangezogen, da bei diesen ein hoher Total-T4 -Gehalt vorliegt. Die beiden Korrelationen zeigen, daß keine direkte Beziehung zwischen dem Gehalt an freiem T4 und dem Gesamt-T4 -Gehalt besteht. Die Korrelationen zeigen ferner, daß der T4/TBG-Quotient in erster Näherung mit dem Verlauf des freien T4 konform geht.

## Patentansprüche (Patentansprüche für folgende(n) Vertragsstaat(en): BE, CH, LI, DE, FR, GB, IT, NL, SE)

1. Verbindung der Formel I 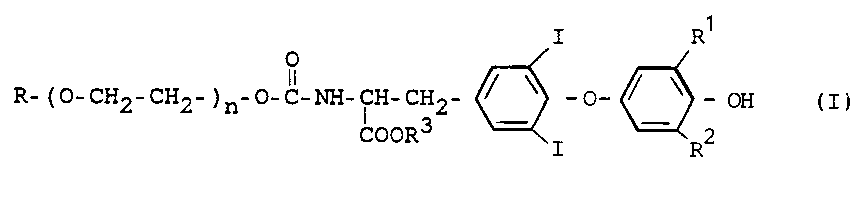 in welcher
R Wasserstoff, (C₁-C₆)-Alkyl oder einen Rest der Formel 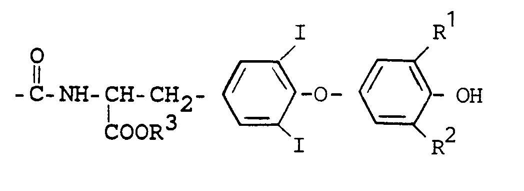 bedeutet,
R¹ und R² gleich oder verschieden sind und Iod oder Wasserstoff bedeuten,
R³ Wasserstoff oder (C₁-C₆)-Alkyl bedeutet und
n für eine ganze Zahl zwischen 10 und 400 steht,
sowie deren physiologisch verträglichen Salze mit Kationen.

2. Verbindung der Formel I gemäß Anspruch 1, in denen n= 35 - 70 ist, R = H oder (C₁-C₄)-Alkyl, und R³ Wasserstoff oder (C₁-C₄)-Alkyl bedeuten.

3. Verbindung der Formel I gemäß Anspruch 1 oder 2 in denen R³ Wasserstoff, Methyl oder Ethyl bedeutet.

4. Verbindung der Formel I gemäß einem der Ansprüche 1-3, in welcher mindestens eines der Iodatome radioaktiv markiert ist.

5. Verbindung der Formel I, in welcher R¹, R², R³ und n wie im Anspruch 1 definiert sind und R für eine mit chemischen oder physikalischen Methoden quantifizierbare Gruppe steht.

6. Verfahren zur Herstellung einer Verbindung der Formel I gemäß Anspruch I, dadurch gekennzeichnet, daß man in einer Verbindung der Formel II
R - (O-CH₂-CH₂-)ₙ -OH (II)
in welcher R und n wie im Anspruch 1 definiert sind, die freie alkoholische OH-Gruppe entweder als Kohlensäureesterchlorid oder als mit geeigneten Aktivestergruppen substituierten Ester der Kohlensäure mit einer Verbindung der Formel III 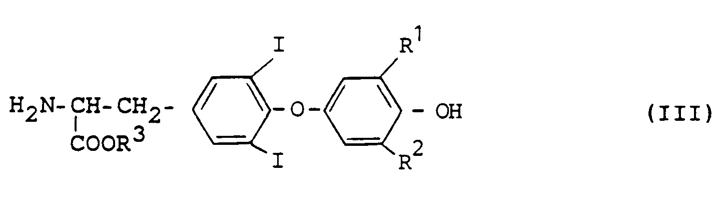 in welcher R¹ und R² wie im Anspruch 1 definiert sind und R³ Wasserstoff bedeutet, umsetzt, oder gegebenenfalls einen Ester der Formel III (R³ ‡ H) einsetzt, erhaltene Ester der Formel I (R³ ‡ H) gegebenenfalls in die freien Säuren der Formel I (R³ = H) überführt, und gegebenenfalls die so erhaltene Verbindung der Formel I in ihre Salze überführt.

7. Verwendung einer Verbindung gemäß einem dieser Ansprüche 1-5 bei der Durchführung eines Immunoassays.

8. Zubereitung enthaltend zwei oder mehrere Verbindungen gemäß den Ansprüchen 1-5.

## Patentansprüche (Patentansprüche für folgende(n) Vertragsstaat(en): ES, AT)

1. Verfahren zur Herstellung einer Verbindung der Formel I 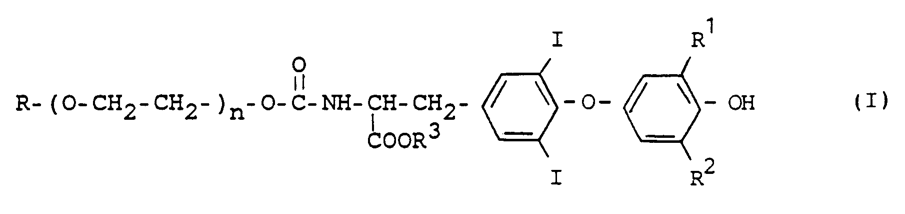 in welcher
R Wasserstoff, (C₁-C₆)-Alkyl oder einen Rest der Formel 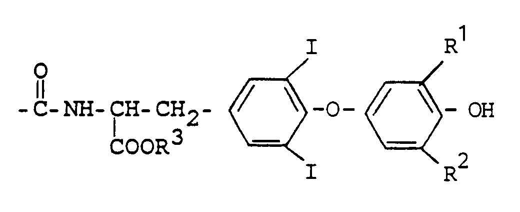 bedeutet,
R¹ und R² gleich oder verschieden sind und Iod oder Wasserstoff bedeuten,
R³ Wasserstoff oder (C₁-C₆)-Alkyl bedeutet und
n für eine ganze Zahl zwischen 10 und 400 steht,
sowie deren physiologisch verträglichen Salze mit Kationen, dadurch gekennzeichnet, daß man in einer Verbindung der Formel II
R -(OCH₂-CH₂- )ₙ- OH (II)
in welcher R und n wie oben definiert sind, die freie Alkoholische OH-Gruppe entweder als Kohlensäureesterchlorid oder als mit geeigneten Aktivestergruppen substituierten Ester der Kohlensäure mit einer Verbindung der Formel III 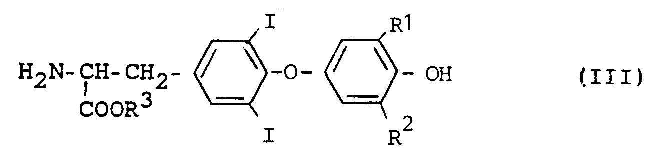 in welcher R¹ und R² wie oben definiert sind und R³ Wasserstoff bedeutet, umsetzt, oder gegebenenfalls einen Ester der Formel III (R³ ‡ H) einsetzt, erhaltene Ester der Formel I (R³ ‡ H), gegebenenfalls in die freien Säuren der Formel I (R³ = H) überführt, und gegebenenfalls die so erhaltene Verbindung der Formel I in ihre Salze überführt.

2. Verfahren zur Herstellung einer Verbindung der Formel I gemäß Anspruch 1, in denen n= 35 - 70 ist, R = H oder (C₁-C₄)-Alkyl, und R³ Wasserstoff oder (C₁-C₄)-Alkyl bedeuten.

3. Verfahren zur Herstellung einer Verbindung der Formel I gemäß Anspruch 1 oder 2 in denen R³ Wasserstoff, Methyl oder Ethyl bedeutet.

4. Verfahren zur Herstellung einer Verbindung der Formel I gemäß einem der Ansprüche 1-3, in welcher mindestens eines der Iodatome radioaktiv markiert ist.

5. Verfahren zur Herstellung einer Verbindung der Formel I, in welcher R¹, R², R³ und n wie im Anspruch 1 definiert sind und für R eine mit chemischen oder physikalischen Methoden quantifizierbare Gruppe steht.

## Claims (Claims for the following Contracting State(s): BE, CH, LI, DE, FR, GB, IT, NL, SE)

1. A compound of the formula I 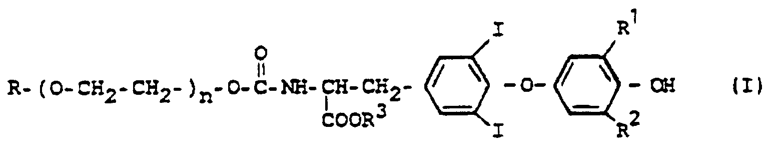 in which
R denotes hydrogen, (C₁-C₆)-alkyl or a radical of the formula 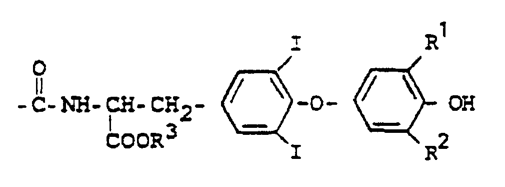
R¹ and R² are identical or different and denote iodine or hydrogen,
R³ denotes hydrogen or (C₁-C₆)-alkyl, and
n represents an integer between 10 and 400,
and the physiologically acceptable salts thereof with cations.

2. A compound of the formula I as claimed in claim 1, in which n is 35 - 70, R denotes H or (C₁-C₄)-alkyl, and R³ denotes hydrogen or (C₁-C₄)-alkyl.

3. A compound of the formula I as claimed in claim 1 or 2, in which R³ denotes hydrogen, methyl or ethyl.

4. A compound of the formula I as claimed in any one of claims 1-3, in which at least one of the iodine atoms is radioactively labeled.

5. A compound of the formula I, in which R¹, R², R³ and n are as defined in claim 1, and R represents a group which can be quantified by chemical or physical methods.

6. A process for preparing a compound of the formula I as claimed in claim 1, wherein, in a compound of the formula II
R - (O-CH₂-CH₂-)ₙ-OH (II)
in which R and n are as defined in claim 1, the free alcoholic OH group is reacted, either as carbonic acid ester chloride or as esters of carbonic acid substituted by suitable active ester groups, with a compound of the formula III 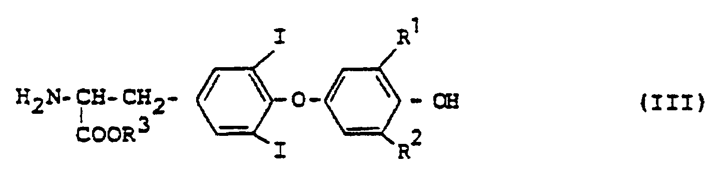 in which R¹ and R² are as defined in claim 1 and R³ denotes hydrogen, or, if appropriate, an ester of the formula III (R³ ≠ H) is employed, the resultant esters of the formula I (R³ ≠ H) are converted, if appropriate, into the free acids of the formula I (R³ = H), and, if appropriate, the compound of the formula I thus obtained is converted into its salts.

7. The use of a compound as claimed in any one of claims 1-5 when carrying out an immunoassay.

8. A preparation comprising two or more compounds as claimed in claims 1-5.

## Claims (Claims for the following Contracting State(s): ES, AT)

1. A process for preparing a compound of the formula I 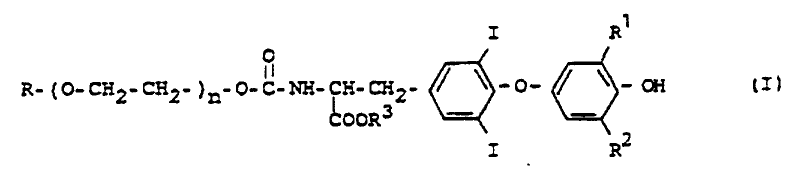 in which
R denotes hydrogen, (C₁-C₆)-alkyl or a radical of the formula 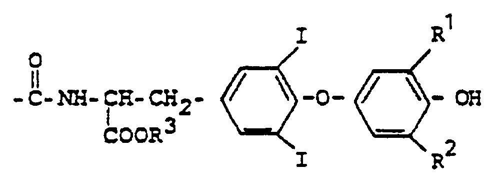
R¹ and R² are identical or different and denote iodine or hydrogen,
R³ denotes hydrogen or (C₁-C₆)-alkyl, and
n represents an integer between 10 and 400,
and the physiologically acceptable salts thereof with cations, wherein, in a compound of the formula II
R - (OCH₂-CH₂-)ₙ-OH (II)
in which R and n are as defined above, the free alcoholic OH group is reacted, either as carbonic acid ester chloride or as esters of carbonic acid substituted by suitable active ester groups, with a compound of the formula III 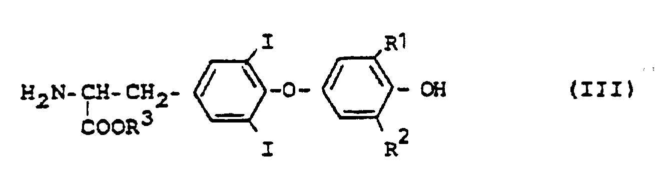 in which R¹ and R² are as defined above and R³ denotes hydrogen, or, if appropriate, an ester of the formula III (R³ ≠ H) is employed, the resultant esters of the formula I (R³ ≠ H) are converted, if appropriate, into the free acids of the formula I (R³ = H), and, if appropriate, the compound of the formula I thus obtained is converted into its salts.

2. The process for preparing a compound of the formula I as claimed in claim 1, in which n is 35 - 70, R denotes H or (C₁-C₄)-alkyl, and R³ denotes hydrogen or (C₁-C₄)-alkyl.

3. The process for preparing a compound of the formula I as claimed in claim 1 or 2, in which R³ denotes hydrogen, methyl or ethyl.

4. The process for preparing a compound of the formula I as claimed in any one of claims 1-3, in which at least one of the iodine atoms is radioactively labeled.

5. The process for preparing a compound of the formula I, in which R¹, R², R³ and n are as defined in claim 1, and R represents a group which can be quantified by chemical or physical methods.

## Revendications (Revendications pour l'(les) Etat(s) contractant(s) suivant(s): BE, CH, LI, DE, FR, GB, IT, NL, SE)

1. Composé de formule I 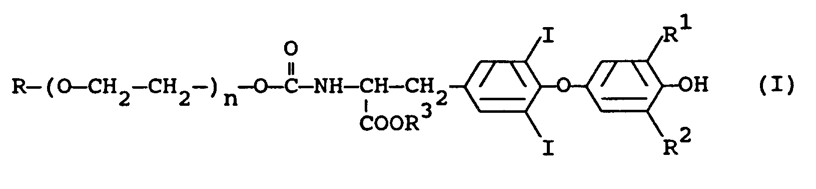 dans laquelle
R représente un atome d'hydrogène ou un groupe alkyle en C₁-C₆ ou un radical de formule 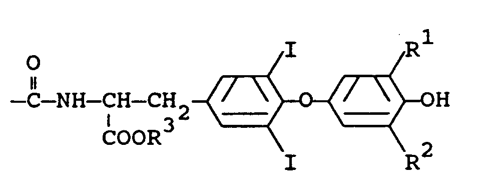
R¹ et R² sont identiques ou différents et représentent un atome d'iode ou d'hydrogène,
R³ représente un atome d'hydrogène ou un groupe alkyle en C₁-C₆, et
n représente un nombre entier compris entre 10 et 400,
et sels physiologiquement acceptables de celui-ci avec des cations.

2. Composé de formule I selon la revendication 1, dans lequel n = 35-70, R = H ou un groupe alkyle en C₁-C₄ et R³ représente un atome d'hydrogène ou un groupe alkyle en C₁-C₄.

3. Composé de formule I selon la revendication 1 ou 2, dans lequel R³ représente un atome d'hydrogène ou le groupe méthyle ou éthyle.

4. Composé de formule I selon l'une des revendications 1 à 3, dans lequel au moins l'un des atomes d'iode est radiomarqué.

5. Composé de formule I dans lequel R¹, R², R³ et n sont tels que définis dans la revendication 1, et R représente un groupe déterminable quantitativement par des méthodes physiques ou chimiques.

6. Procédé pour la préparation d'un composé de formule I selon la revendication 1, caractérisé en ce que, dans un composé de formule II
R-(O-CH₂-CH₂-)ₙ-OH (II)
dans laquelle R et n sont tels que définis dans la revendication 1, on fait réagir le groupe alcoolique OH libre, soit sous forme de chlorure d'ester carboxylique, soit sous forme d'ester de l'acide carboxylique, substitué par des groupes esters activés appropriés, avec un composé de formule III 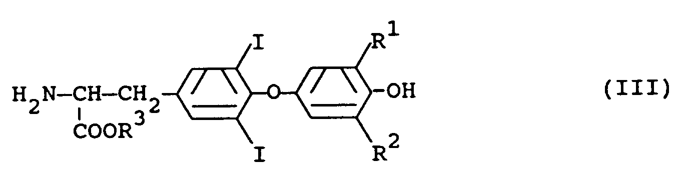 dans laquelle R¹ et R² sont tels que définis dans la revendication 1, et R³ représente un atome d'hydrogène, ou éventuellement on utilise un ester de formule III (R³ ≠ H), on convertit éventuellement des esters de formule I (R³ ≠ H) obtenus en les acides libres de formule I (R³ = H) et éventuellement on convertit le composé de formule I ainsi obtenu en ses sels.

7. Utilisation d'un composé selon l'une des revendications 1 à 5, dans l'exécution d'un essai immunologique.

8. Composition contenant deux composés ou plus selon les revendications 1 à 5.

## Revendications (Revendications pour l'(les) Etat(s) contractant(s) suivant(s): ES, AT)

1. Procédé pour la préparation d'un composé de formule I 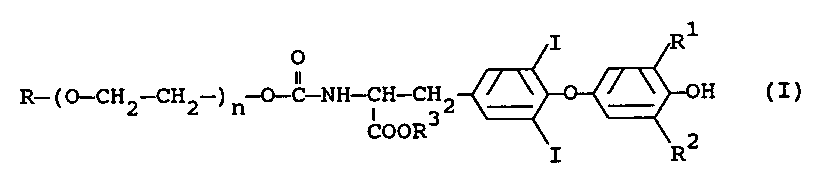 dans laquelle
R représente un atome d'hydrogène ou un groupe alkyle en C₁-C₆ ou un radical de formule 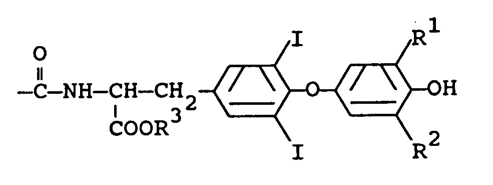
R¹ et R² sont identiques ou différents et représentent un atome d'iode ou d'hydrogène,
R³ représente un atome d'hydrogène ou un groupe alkyle en C₁-C₆, et
n représente un nombre entier compris entre 10 et 400,
et de ses sels physiologiquement acceptables avec des cations, caractérisé en ce que, dans un composé de formule II
R-(O-CH₂-CH₂-)ₙ-OH (II)
dans laquelle R et n sont tels que définis plus haut, on fait réagir le groupe alcoolique OH libre, soit sous forme de chlorure d'ester carboxylique,soit sous forme d'ester de l'acide carboxylique, substitué par des groupes esters activés appropriés, avec un composé de formule III 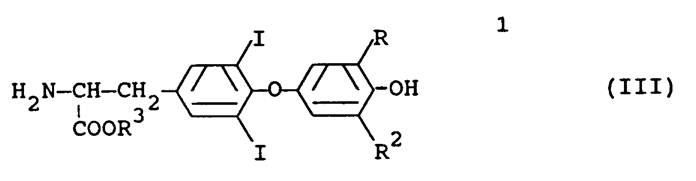 dans laquelle R¹ et R² sont tels que définis plus haut, et R³ représente un atome d'hydrogène, ou éventuellement on utilise un ester de formule III (R³ ≠ H), on convertit éventuellement des esters de formule I (R³ ≠ H) obtenus en les acides libres de formule I (R³ = H) et éventuellement on convertit le composé de formule I ainsi obtenu en ses sels.

2. Procédé pour la préparation d'un composé de formule I selon la revendication 1, dans lequel n = 35-70, R = H ou un groupe alkyle en C₁-C₄ et R³ représente un atome d'hydrogène ou un groupe alkyle en C₁-C₄.

3. Procédé pour la préparation d'un composé de formule I selon la revendication 1 ou 2, dans lequel R³ représente un atome d'hydrogène ou le groupe méthyle ou éthyle.

4. Procédé pour la préparation d'un composé de formule I selon l'une des revendications 1 à 3, dans lequel au moins l'un des atomes d'iode est radiomarqué.

5. Procédé pour la préparation d'un composé de formule I dans lequel R¹, R², R³ et n sont tels que définis dans la revendication 1, et R représente un groupe déterminable quantitativement par des méthodes physiques ou chimiques.
